# EUROPEAN PATENT APPLICATION

(11) **EP 4 230 163 A1**
(43) Date of publication of application: **23.08.2023**
(21) Application number: 23155588.9
(22) Date of filing: 08.02.2023
(51) Int. Cl.: A61B 18/14, A61B 18/12, A61B 18/00

(54) **SURGICAL TOOL WITH PROXIMAL THERMOCOUPLE**

(30) Priority: 17.02.2022 US 202217674462
(71) Applicant: Medtronic Holding Company Sàrl, 1131 Tolochenaz (CH)
(72) Inventor: NORTON, Jeffrey A., Sunnyvale, 94085 (US)
(74) Representative: Zimmermann & Partner Patentanwälte mbB

(57) **Abstract**

A surgical tool according to at least one embodiment of the present disclosure includes a proximal end; and a distal end, the distal end including a first electrode; a second electrode different from the first electrode; and a thermal bridge that is disposed between the first electrode and the second electrode and that electrically isolates the first electrode from the second electrode.

## Description

### BACKGROUND

The present disclosure is generally directed to surgical tools and relates more particularly to surgical tools providing ablation capabilities.

Various surgical tools are often required to successfully complete surgical procedures. Certain types of tools may be required for different procedures. Some surgical tools utilize applied electrical current for ablation.

### BRIEF SUMMARY

Example aspects of the present disclosure include:

A surgical tool according to at least one embodiment of the present disclosure comprises: a proximal end; and a distal end, the distal end comprising: a first electrode; a second electrode different from the first electrode; and a thermal bridge that is disposed between the first electrode and the second electrode and that electrically isolates the first electrode from the second electrode.

Any of the aspects herein, wherein the distal end further comprises: a thermocouple disposed on a proximal end of the first electrode.

Any of the aspects herein, wherein the first electrode comprises an active Radio Frequency (RF) electrode, and wherein the second electrode comprises a return RF electrode.

Any of the aspects herein, wherein the thermal bridge is at least one of alumina or artificial diamond.

Any of the aspects herein, wherein the first electrode, the second electrode, and the thermal bridge are substantially cylindrical, and wherein the first electrode, the second electrode, and the thermal bridge are aligned coaxially.

Any of the aspects herein, further comprising: an inflow tube disposed in an interior of the surgical tool and extending from the proximal end to a first distance from the distal end, the inflow tube configured to discharge a coolant into a cavity in the distal end of the surgical tool; and an outflow tube disposed in the interior of the surgical tool and extending from the proximal end to the first distance from the distal end, the outflow tube configured to remove the coolant from the cavity in the distal end of the surgical tool.

Any of the aspects herein, wherein the first distance from the distal end is within an interior of the thermal bridge.

Any of the aspects herein, wherein the thermal bridge comprises one or more interlocking keys, and wherein the one or more interlocking keys connect the thermal bridge to the first electrode and to the second electrode.

Any of the aspects herein, wherein the thermal bridge is soldered to at least one of the first electrode or the second electrode.

Any of the aspects herein, wherein the second electrode comprises a domed tip, and wherein the surgical tool further comprises: an active RF wire extending through an interior of the surgical tool and connected to the domed tip.

A surgical system according to at least one embodiment of the present disclosure comprises: a cylindrical housing having a proximal end and a distal end; a proximal tube, a first end of the proximal tube connected to a first end of the distal end; a first thermocouple disposed between at least a portion of a return wire and the proximal tube; a heat sink, a first end of the heat sink connected to a second end of the proximal tube; and a distal tube, a first end of the distal tube connected to a second end of the heat sink.

Any of the aspects herein, further comprising: an inflow conduit disposed in the cylindrical housing and configured to dispense a coolant into a cavity within the distal tube; and an outflow conduit disposed in the cylindrical housing and configured to extract the coolant from the cavity within the distal tube.

Any of the aspects herein, wherein at least one of a first end of the inflow conduit or a first end of the outflow conduit is disposed in the heat sink, and wherein the cylindrical housing comprises an air gap between the cavity and the distal tube.

Any of the aspects herein, wherein the surgical system further comprises: a polyimide insulation layer disposed around at least one of the cylindrical housing or the return wire.

Any of the aspects herein, wherein the heat sink comprises at least one of alumina or artificial diamond.

Any of the aspects herein, wherein the cylindrical housing further comprises: an active Radiofrequency (RF) wire extending from the proximal end of the cylindrical housing to a domed tip disposed in the distal tube, the active RF wire configured to carry current to the distal tube.

Any of the aspects herein, wherein the heat sink is soldered to at least one of the proximal tube or the distal tube.

An apparatus according to at least one embodiment of the present disclosure comprises: a fluid management system including an inflow tube and an outflow tube; and a surgical probe, the surgical probe comprising: an active Radiofrequency (RF) electrode; a return RF electrode; and a distal bridge that physically and thermally couples the return RF electrode and the active RF electrode and electrically decouples the return RF electrode and the active RF electrode.

Any of the aspects herein, wherein at least one of the inflow tube and the outflow tube is in fluidic communication with the surgical probe, and wherein at least one of the inflow tube and the outflow tube ends within the distal bridge.

Any of the aspects herein, wherein the distal bridge comprises at least one of alumina or artificial diamond, and wherein the surgical probe further comprises: a thermocouple disposed on a proximal end of the return RF electrode, wherein the thermocouple generates a temperature reading of the return RF electrode.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Use of any one or more of the aspects or features as disclosed herein.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

Further disclosed herein is a surgical tool according to at least one embodiment of the present disclosure wherein the surgical tool includes a proximal end; and a distal end, the distal end including a first electrode; a second electrode different from the first electrode; and a thermal bridge that is disposed between the first electrode and the second electrode and that electrically isolates the first electrode from the second electrode.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1A is a block diagram of aspects of a system according to at least one embodiment of the present disclosure;
Fig. 1B is a block diagram of aspects of a system according to at least one embodiment of the present disclosure;
Fig. 2A is a perspective view of a surgical tool according to at least one embodiment of the present disclosure;
Fig. 2B is a detailed view of a distal end of the surgical tool according to at least one embodiment of the present disclosure;
Fig. 2C is a detailed view of an interior of the distal end of the surgical tool according to at least one embodiment of the present disclosure;
Fig. 2D is a detailed view of an interior of a thermal bridge of the surgical tool according to at least one embodiment of the present disclosure;
Fig. 2E is a perspective view of a proximal end of the surgical tool according to at least one embodiment of the present disclosure;
Fig. 3 is a perspective view of a surgical tool being used in a surgery or surgical procedure according to at least one embodiment of the present disclosure; and
Fig. 4 is a flowchart according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system.

The growth of tumors within and around the spine may cause periostitis pain and vertebrogenic pain and are common causes of debilitating back pain experienced by patients afflicted with, for example, late stage metastatic cancers. Radiofrequency (RF) energy may be applied to destroy the tumor and may be used during the course of palliative treatment for such patients.

In some embodiments, probes may be used to apply the RF energy. The probes may be cooled probes with thermocouples disposed distally or non-cooled probes with thermocouples disposed proximally. The cooled probes may be capable of actively cooling the electrodes, with the cooling inhibiting charring of tissues adjacent to where the tumor or lesion is growing. This permits for greater lesion sizes to be created by the RF probe at greater rates relative to non-cooled probes. The thermocouple disposed at a distal end or tip may provide measurements or other readings to a physician about the temperature of the tissues at the distal end of the probe. However, such thermocouple placement may make it difficult to monitor the temperature of electrodes on the proximal end of the surgical tool, which may be near critical structures of the spine.

In cooled probes, design constraints may make it difficult to dispose the thermocouple on the proximal side because the cooling of the RF electrodes may bias the readings of the thermocouple toward lower temperatures, which may result in inaccuracies in determining tissue and lesion sizes.

Non-cooled probes often generate smaller lesion sizes at slower rates relative to cooled probes. However, non-cooled probes have the advantage of being able to have thermocouples disposed on the surgical tool (e.g., to the proximal end of a proximal electrode) to more accurately map and/or measure the temperature of the lesion size formed by the RF probe.

In at least one embodiment of the present disclosure, an RF probe combines the efficacy of a cooled probe with a thermocouple disposed proximal relative the electrodes.

In at least one embodiment of the present disclosure, the active electrode may be disposed distally and/or coaxially to the return electrode. The distal thermocouple may provide an electrical connection for the active electrode that is isolated (e.g., physically, electrically, etc.) from the return electrode via a dielectric layer surrounding the distal thermocouple. Additionally, a reflective carrier tube (e.g., made of stainless steel or other metal) may surround the distal thermocouple. In at least one embodiment, the return and active electrodes, may be or comprise stainless steel, may be separated by a first distance, and may be electrically isolated from each other by an electrically isolating bridge disposed between the active electrode and the return electrode.

A coolant (e.g., sterile water) may be flowed into the distal area of the probe through isolated stainless steel tube channels for inflow and outflow. In some embodiments, the inflow and outflow tubes may be adjacent to one another. The channels through which the coolant flows may be electrically isolated from the return electrode. For instance, the channels may end within the isolating bridge, such that neither tube contacts nor comes into electrical connection with the distal electrode (e.g., the active electrode). The positioning of the coolant channels relative to the isolating bridge may cool the bridge. The bridge may be or comprise material with a high thermal conductivity such as alumina ceramic. The alumina may conduct heat from the active and return electrodes between which the alumina is connected. While the return electrode may be isolated from direct contact with the coolant, the return electrode is attached to the bridge, and is therefore still cooled by the coolant flow through thermal conduction (e.g., the return electrode heat is transferred to the alumina, and the heat moves from the alumina to the coolant). By keeping the electrodes thermally isolated from the coolant, a thermocouple disposed proximal both electrodes is capable of generating more accurate temperature measurements of the lesion proximal the electrodes.

In some embodiments, insulation may be disposed around one or more components of the surgical tool. For instance, insulation may be placed between a carrier tube (e.g., a conduit containing an active current-carrying wire) and the active wire. Such insulation may reduce or prevent capacitive coupling (e.g., induction of current to the carrier tube), improving physician and/or patient safety. Capacitive coupling contributes to tube heating (e.g., increased heating of electrodes or other components of the surgical tool), energy loss, and ambiguous power delivery to the patient. The coupling may also cause power delivery to locations outside of the lesion zone. Such capacitive coupling may be address with the placement of insulation between the wire and the carrier tube.

The application of the above embodiments is not particularly limited, and ablation of any surgical site using the above techniques may be possible, such as ablation of spinal elements, as well as other ablations outside the spine. Further, the above techniques may be used to generate smaller or larger lesion sizes.

Use of the foregoing may enable cooling ablation while simultaneously better monitoring of lesion size and temperature, decreasing the time needed to perform the surgery or surgical procedure. The increased accuracy of the readings of the temperatures of the proximal and distal lesion may be displayed real-time (e.g., to a controller/generator screen), reducing patient risk of damage to critical structures (e.g., critical structures in a vertebra). The embodiments discussed herein provide a bridge that provides thermal conduction capabilities during ablation while also maintaining electrical isolation. The separation of the electrodes directly from the coolant improves the accuracy of the thermocouple measurements.

Embodiments of the present disclosure provide technical solutions to one or more of the problems of (1) inaccurate thermocouple readings during ablation, and (2) charring of adjacent tissues to a lesion caused by ablation.

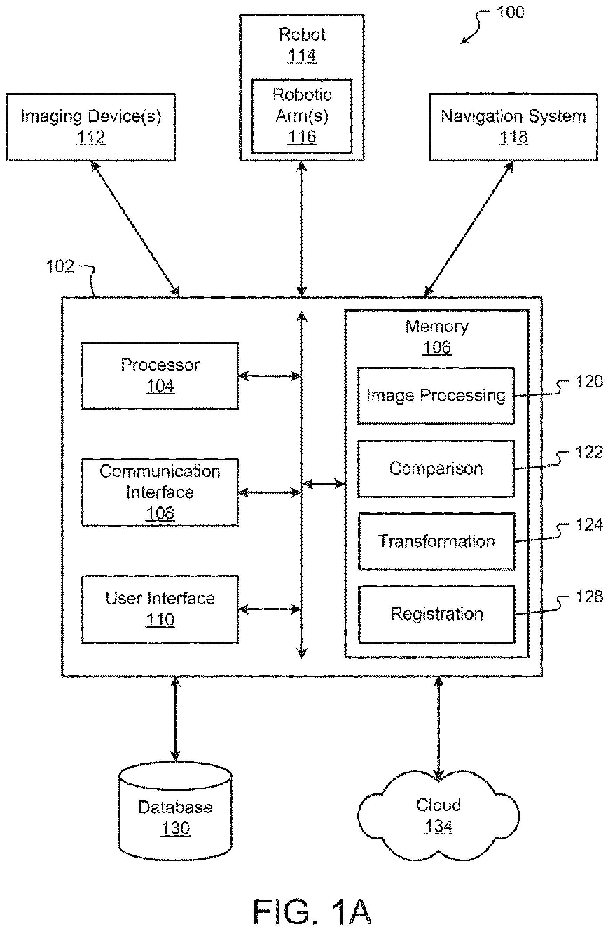

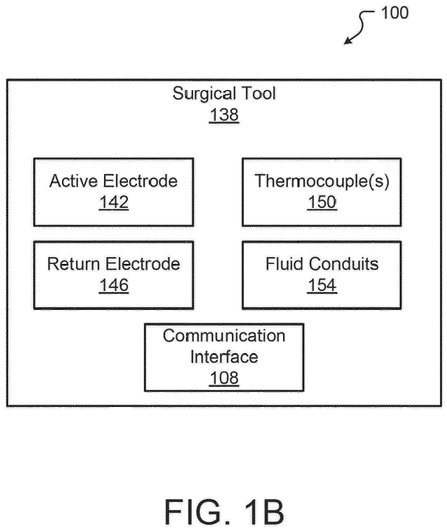

Turning first to Fig. 1, a block diagram of a system 100 according to at least one embodiment of the present disclosure is shown. The system 100 may be used to facilitate ablation of anatomical tissue; to monitor the ablation of the anatomical tissue; to control, pose, and/or otherwise manipulate a surgical mount system, a surgical arm, and/or surgical tools attached thereto; and/or carry out one or more other aspects of one or more of the methods disclosed herein. The system 100 comprises a computing device 102, one or more imaging devices 112, a robot 114, a navigation system 118, a database 130, and/or a cloud or other network 134. Systems according to other embodiments of the present disclosure may comprise more or fewer components than the system 100. For example, the system 100 may not include the imaging device 112, the robot 114, the navigation system 118, one or more components of the computing device 102, the database 130, and/or the cloud 134.

The computing device 102 comprises a processor 104, a memory 106, a communication interface 108, and a user interface 110. Computing devices according to other embodiments of the present disclosure may comprise more or fewer components than the computing device 102.

The processor 104 of the computing device 102 may be any processor described herein or any similar processor. The processor 104 may be configured to execute instructions stored in the memory 106, which instructions may cause the processor 104 to carry out one or more computing steps utilizing or based on data received from the imaging device 112, the robot 114, the navigation system 118, the database 130, and/or the cloud 134.

The memory 106 may be or comprise RAM, DRAM, SDRAM, other solid-state memory, any memory described herein, or any other tangible, non-transitory memory for storing computer-readable data and/or instructions. The memory 106 may store information or data useful for completing, for example, any step of the method 400 described herein, or of any other methods. The memory 106 may store, for example, instructions and/or machine learning models that support one or more functions of the robot 114. For instance, the memory 106 may store content (e.g., instructions and/or machine learning models) that, when executed by the processor 104, enable image processing 120, comparison 122, transformation 124, and/or registration 128. Such content, if provided as in instruction, may, in some embodiments, be organized into one or more applications, modules, packages, layers, or engines. Alternatively or additionally, the memory 106 may store other types of content or data (e.g., machine learning models, artificial neural networks, deep neural networks, etc.) that can be processed by the processor 104 to carry out the various method and features described herein. Thus, although various contents of memory 106 may be described as instructions, it should be appreciated that functionality described herein can be achieved through use of instructions, algorithms, and/or machine learning models. The data, algorithms, and/or instructions may cause the processor 104 to manipulate data stored in the memory 106 and/or received from or via the imaging device 112, the robot 114, the database 130, and/or the cloud 134.

The computing device 102 may also comprise a communication interface 108. The communication interface 108 may be used for receiving image data or other information from an external source (such as the imaging device 112, the robot 114, the navigation system 118, the database 130, the cloud 134, and/or any other system or component not part of the system 100), and/or for transmitting instructions, images, or other information to an external system or device (e.g., another computing device 102, the imaging device 112, the robot 114, the navigation system 118, the database 130, the cloud 134, and/or any other system or component not part of the system 100). The communication interface 108 may comprise one or more wired interfaces (e.g., a USB port, an Ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 108 may be useful for enabling the device 102 to communicate with one or more other processors 104 or computing devices 102, whether to reduce the time needed to accomplish a computing-intensive task or for any other reason.

The computing device 102 may also comprise one or more user interfaces 110. The user interface 110 may be or comprise a keyboard, mouse, trackball, monitor, television, screen, touchscreen, and/or any other device for receiving information from a user and/or for providing information to a user. The user interface 110 may be used, for example, to receive a user selection or other user input regarding any step of any method described herein. Notwithstanding the foregoing, any required input for any step of any method described herein may be generated automatically by the system 100 (e.g., by the processor 104 or another component of the system 100) or received by the system 100 from a source external to the system 100. In some embodiments, the user interface 110 may be useful to allow a surgeon or other user to modify instructions to be executed by the processor 104 according to one or more embodiments of the present disclosure, and/or to modify or adjust a setting of other information displayed on the user interface 110 or corresponding thereto.

Although the user interface 110 is shown as part of the computing device 102, in some embodiments, the computing device 102 may utilize a user interface 110 that is housed separately from one or more remaining components of the computing device 102. In some embodiments, the user interface 110 may be located proximate one or more other components of the computing device 102, while in other embodiments, the user interface 110 may be located remotely from one or more other components of the computing device 102.

The imaging device 112 may be operable to image anatomical feature(s) (e.g., a bone, veins, tissue, etc.) and/or other aspects of patient anatomy to yield image data (e.g., image data depicting or corresponding to a bone, veins, tissue, etc.). "Image data" as used herein refers to the data generated or captured by an imaging device 112, including in a machine-readable form, a graphical/visual form, and in any other form. In various examples, the image data may comprise data corresponding to an anatomical feature of a patient, or to a portion thereof. The image data may be or comprise a preoperative image, an intraoperative image, a postoperative image, or an image taken independently of any surgical procedure. In some embodiments, a first imaging device 112 may be used to obtain first image data (e.g., a first image) at a first time, and a second imaging device 112 may be used to obtain second image data (e.g., a second image) at a second time after the first time. The imaging device 112 may be capable of taking a 2D image or a 3D image to yield the image data. The imaging device 112 may be or comprise, for example, an ultrasound scanner (which may comprise, for example, a physically separate transducer and receiver, or a single ultrasound transceiver), an O-arm, a C-arm, a G-arm, or any other device utilizing X-ray-based imaging (e.g., a fluoroscope, a CT scanner, or other X-ray machine), a magnetic resonance imaging (MRI) scanner, an optical coherence tomography (OCT) scanner, an endoscope, a microscope, an optical camera, a thermographic camera (e.g., an infrared camera), a radar system (which may comprise, for example, a transmitter, a receiver, a processor, and one or more antennae), or any other imaging device 112 suitable for obtaining images of an anatomical feature of a patient. The imaging device 112 may be contained entirely within a single housing, or may comprise a transmitter/emitter and a receiver/detector that are in separate housings or are otherwise physically separated.

In some embodiments, the imaging device 112 may comprise more than one imaging device 112. For example, a first imaging device may provide first image data and/or a first image, and a second imaging device may provide second image data and/or a second image. In still other embodiments, the same imaging device may be used to provide both the first image data and the second image data, and/or any other image data described herein. The imaging device 112 may be operable to generate a stream of image data. For example, the imaging device 112 may be configured to operate with an open shutter, or with a shutter that continuously alternates between open and shut so as to capture successive images. For purposes of the present disclosure, unless specified otherwise, image data may be considered to be continuous and/or provided as an image data stream if the image data represents two or more frames per second.

The robot 114 may be any surgical robot or surgical robotic system. The robot 114 may be or comprise, for example, the Mazor X^{™} Stealth Edition robotic guidance system. The robot 114 may be configured to position the imaging device 112 at one or more precise position(s) and orientation(s), and/or to return the imaging device 112 to the same position(s) and orientation(s) at a later point in time. The robot 114 may additionally or alternatively be configured to manipulate a surgical tool (whether based on guidance from the navigation system 118 or not) to accomplish or to assist with a surgical task. In some embodiments, the robot 114 may be configured to hold and/or manipulate an anatomical element during or in connection with a surgical procedure. The robot 114 may comprise one or more robotic arms 116. In some embodiments, the robotic arm 116 may comprise a first robotic arm and a second robotic arm, though the robot 114 may comprise more than two robotic arms. In some embodiments, one or more of the robotic arms 116 may be used to hold and/or maneuver the imaging device 112. In embodiments where the imaging device 112 comprises two or more physically separate components (e.g., a transmitter and receiver), one robotic arm 116 may hold one such component, and another robotic arm 116 may hold another such component. Each robotic arm 116 may be positionable independently of the other robotic arm. The robotic arms 116 may be controlled in a single, shared coordinate space, or in separate coordinate spaces.

The robot 114, together with the robotic arm 116, may have, for example, one, two, three, four, five, six, seven, or more degrees of freedom. Further, the robotic arm 116 may be positioned or positionable in any pose, plane, and/or focal point. The pose includes a position and an orientation. As a result, an imaging device 112, surgical tool, or other object held by the robot 114 (or, more specifically, by the robotic arm 116) may be precisely positionable in one or more needed and specific positions and orientations.

The robotic arm(s) 116 may comprise one or more sensors that enable the processor 104 (or a processor of the robot 114) to determine a precise pose in space of the robotic arm (as well as any object or element held by or secured to the robotic arm).

In some embodiments, reference markers (e.g., navigation markers) may be placed on the robot 114 (including, e.g., on the robotic arm 116), the imaging device 112, or any other object in the surgical space. The reference markers may be tracked by the navigation system 118, and the results of the tracking may be used by the robot 114 and/or by an operator of the system 100 or any component thereof. In some embodiments, the navigation system 118 can be used to track other components of the system (e.g., imaging device 112) and the system can operate without the use of the robot 114 (e.g., with the surgeon manually manipulating the imaging device 112 and/or one or more surgical tools, based on information and/or instructions generated by the navigation system 118, for example).

The navigation system 118 may provide navigation for a surgeon and/or a surgical robot during an operation. The navigation system 118 may be any now-known or future-developed navigation system, including, for example, the Medtronic StealthStation^{™} S8 surgical navigation system or any successor thereof. The navigation system 118 may include one or more cameras or other sensor(s) for tracking one or more reference markers, navigated trackers, or other objects within the operating room or other room in which some or all of the system 100 is located. The one or more cameras may be optical cameras, infrared cameras, or other cameras. In some embodiments, the navigation system 118 may comprise one or more electromagnetic sensors. In various embodiments, the navigation system 118 may be used to track a position and orientation (e.g., a pose) of the imaging device 112, the robot 114 and/or robotic arm 116, and/or one or more surgical tools (or, more particularly, to track a pose of a navigated tracker attached, directly or indirectly, in fixed relation to the one or more of the foregoing). The navigation system 118 may include a display for displaying one or more images from an external source (e.g., the computing device 102, imaging device 112, or other source) or for displaying an image and/or video stream from the one or more cameras or other sensors of the navigation system 118. In some embodiments, the system 100 can operate without the use of the navigation system 118. The navigation system 118 may be configured to provide guidance to a surgeon or other user of the system 100 or a component thereof, to the robot 114, or to any other element of the system 100 regarding, for example, a pose of one or more anatomical elements, whether or not a tool is in the proper trajectory, and/or how to move a tool into the proper trajectory to carry out a surgical task according to a preoperative or other surgical plan.

The database 130 may store information that correlates one coordinate system to another (e.g., one or more robotic coordinate systems to a patient coordinate system and/or to a navigation coordinate system). The database 130 may additionally or alternatively store, for example, one or more surgical plans (including, for example, pose information about a target and/or image information about a patient's anatomy at and/or proximate the surgical site, for use by the robot 114, the navigation system 118, and/or a user of the computing device 102 or of the system 100); one or more images useful in connection with a surgery to be completed by or with the assistance of one or more other components of the system 100; and/or any other useful information. The database 130 may be configured to provide any such information to the computing device 102 or to any other device of the system 100 or external to the system 100, whether directly or via the cloud 134. In some embodiments, the database 130 may be or comprise part of a hospital image storage system, such as a picture archiving and communication system (PACS), a health information system (HIS), and/or another system for collecting, storing, managing, and/or transmitting electronic medical records including image data.

The cloud 134 may be or represent the Internet or any other wide area network. The computing device 102 may be connected to the cloud 134 via the communication interface 108, using a wired connection, a wireless connection, or both. In some embodiments, the computing device 102 may communicate with the database 130 and/or an external device (e.g., a computing device) via the cloud 134.

The system 100 includes a surgical tool 138. The surgical tool 138 may be adapted to, among other things, perform a surgery or surgical operation on one or more anatomical elements (e.g., vertebrae), and can communicate with the computing device 102, the processor 104, and/or any other component of the system 100 via, for example, the communication interface 108. For instance, the surgical tool 138 may be operated or controlled autonomously (e.g., the computing device 102 controls the surgical tool 138, which may be connected to a robotic arm 116 whose pose is also controlled by the computing device 102) or semi-autonomously (e.g., a user such as a surgeon controls the positioning of the surgical tool 138, while the computing device 102 controls one or more operations of the tool such as coolant flow rates). In some embodiments, the system 100 may comprise more than one surgical tool 138. The surgical tool 138 comprises an active electrode 142, a return electrode 146, one or more thermocouples 150, and fluid conduits 154. Notwithstanding the foregoing, surgical tools discussed herein are in no way limited to these components, and additional or alternative components may be present.

The active electrode 142 and the return electrode 146 may enable ablation of anatomical elements (or portions thereof) or, more generally, anatomical tissue using RF energy. For instance, a distal end of the surgical tool 138 may be placed in a surgical site, such that the active electrode 142 and the return electrode 146 contact the anatomical tissue to be treated. The surgical tool 138 may then generate RF energy that enters the anatomical tissue from the active electrode 142, passes through the anatomical tissue, and exits through the return electrode 146. The completion of the circuit causes the anatomical tissue to conduct the current passing between the electrodes 142, 146, heating the anatomical tissue. In such embodiments, the surgical tool 138 utilizes bipolar ablation, where each of the electrodes 142, 146 acts as a pole to conduct current therebetween (e.g., through the adjacent anatomical tissue). The active electrode 142 and/or the return electrode 146 may be made of or comprise conductive material (e.g., metals such as stainless steel), and may be spaced a first distance from one another.

In some embodiments, the active electrode 142 and the return electrode 146 may be or comprise rectangular cylinders, or may be otherwise cylindrically shaped. In such embodiments, the active electrode 142 and the return electrode 146 may be coaxially aligned. In other words, the active electrode 142 and/or the return electrode 146 may be aligned with one another along an axis. In still further embodiments, the active electrode 142 and the return electrode 146 may have similar or the same radii. In one embodiment, the active electrode 142 and the return electrode 146 may be coaxially aligned with substantially the same radii.

The surgical tool 138 also comprises one or more thermocouples 150. The thermocouples 150 may be or comprise sensors that measure the temperature of the active electrode 142 and/or the return electrode 146. Each of the electrodes 142, 146 may be monitored by a separate thermocouple. For instance, a first thermocouple may be disposed proximate the active electrode 142 and a second thermocouple may be disposed near the return electrode 146. The sensors may, based on the heating experienced by the thermocouples 150 (e.g., from heat generated by the electrodes 142, 146), generate measurements indicative of the temperature of the thermocouple (and, by extension, the temperature of the active electrode 142 and/or the return electrode 146).

The surgical tool 138 includes one or more fluid conduits 154. The fluid conduits 154 may channel a coolant through the surgical tool 138 to cool the active electrode 142, the return electrode 146, and/or other components of the surgical tool 138. The coolant may be water, sterilized water, saline, combinations thereof, or the like. The fluid conduits 154 may be connected to a fluid reservoir, which may be controlled (e.g., by a surgeon, by the computing device 102, etc.) to pump the fresh coolant into the surgical tool 138 and remove spent coolant therefrom. In some embodiments, the reservoir may contain separate sub-volumes for the coolant entering the surgical tool 138 and for coolant exiting the surgical tool 138.

The system 100 or similar systems may be used, for example, to carry out one or more aspects of the method 400 described herein. The system 100 or similar systems may also be used for other purposes.

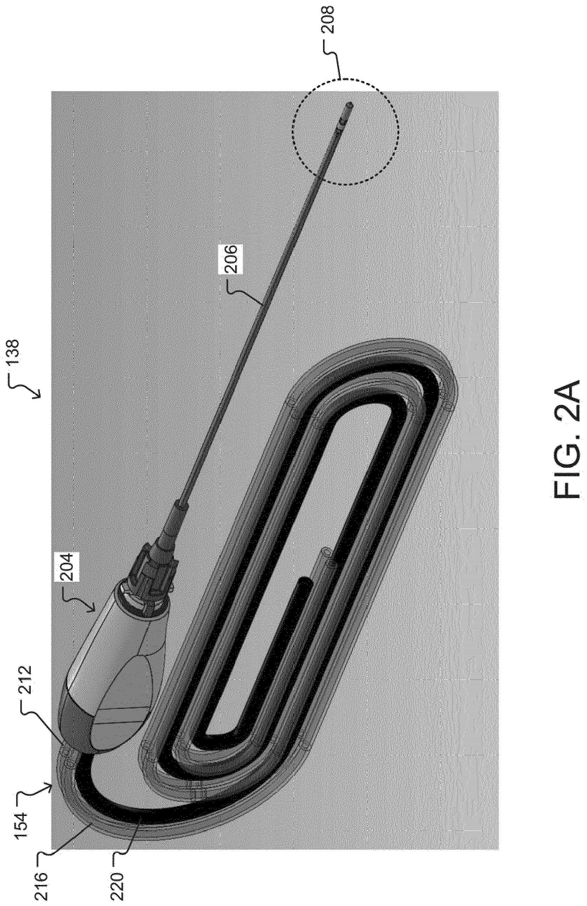

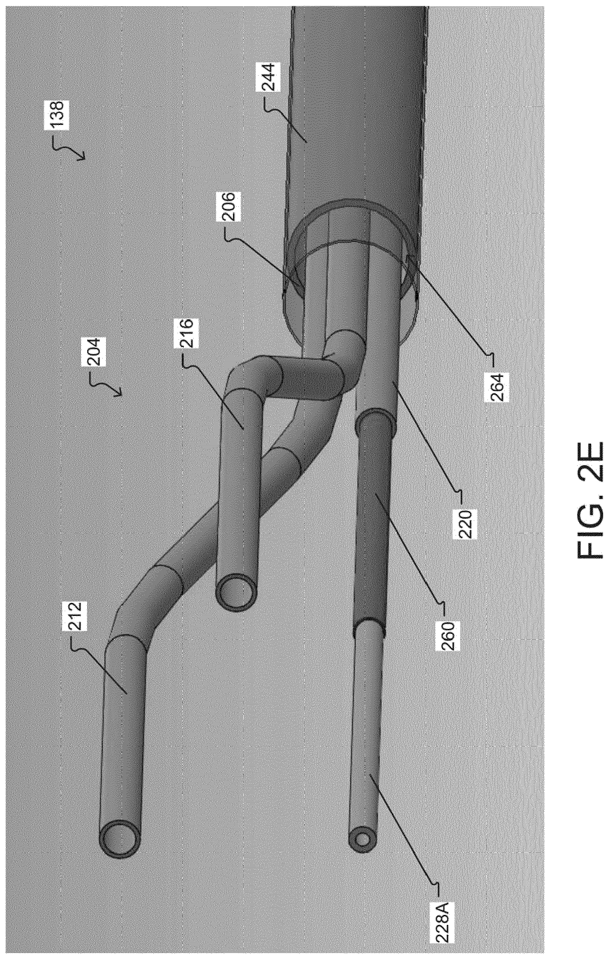

Turning now to Figs. 2A-2E, aspects of the surgical tool 138 are depicted in accordance with at least one embodiment of the present disclosure. The surgical tool 138 includes a proximal end 204, an elongated shaft 206, and a distal end 208. The proximal end 204 may be positioned closer to a user (e.g., a surgeon) and may not interact with the surgical site, while the distal end 208 is further from the user and may interact with the surgical site. The elongated shaft 206 may span between the distal end 208 and the proximal end 204 and may enable the distal end 208 to enter the surgical site (e.g., an interior of a vertebra) while also allowing the proximal end 204 to be manipulated by the user. The proximal end 204, the rc206, and/or the distal end 208 may act as a housing for one or more components of the surgical tool 138. For instance, the elongated shaft 206 may be hollow to permit an inflow tube 212, an outflow tube 216, components of the electrical conduit 220 (e.g., current-carrying wires, insulation, etc.), combinations thereof, and/or the like to extend from the proximal end 204 through the elongated shaft 206 to the distal end 208.

The proximal end 204 of the surgical tool 138 may include the fluid conduits 154, which comprise the inflow tube 212 and the outflow tube 216. The inflow tube 212 may be or comprise tubing extending through the proximal end 204, the elongated shaft 206, and the distal end 208 that carries in fresh coolant from a fluid reservoir (not shown) to cool components in of the distal end 208. In some embodiments, the inflow tube 212 and/or the outflow tube 216 may include thermal insulation, such that the coolant experiences reduced or limited heating or cooling before entering the surgical tool 138.

The proximal end 204 of the surgical tool 138 also comprises an electrical conduit 220. The electrical conduit 220 may be an electrically insulated tube that enables current flow to the surgical tool 138. For example, the electrical conduit 220 may include a current-carrying wire that contacts or connects to the distal end 208 of the surgical tool 138, such that the active electrode 142 receives a current carried by the electrical conduit 220. In some embodiments, the electrical conduit 220 may be connected to the proximal end 204 at a first end and be connected to a current-generating device at a second end. The current-carrying wire may carry current from the current-generating device

(e.g., a generator) to the distal end 208 of the surgical tool 138. The amount of current passing through the current-carrying wire may be regulated by the computing device 102, a user, or another controller or regulating device outside the system 100.

In some embodiments, the inflow tube 212, the outflow tube 216, and/or the electrical conduit 220 may be separated from the elongated shaft 206 in a radial direction by an air gap 264. The air gap 264 may prevent the coolant flowing through the inflow tube 212 and/or the outflow tube 216 from interacting with the return current and the return electrode 146, such that a proximal thermocouple can obtain a more accurate temperature measurement of the return electrode 146 and/or the proximal lesion being formed during the RF ablation.

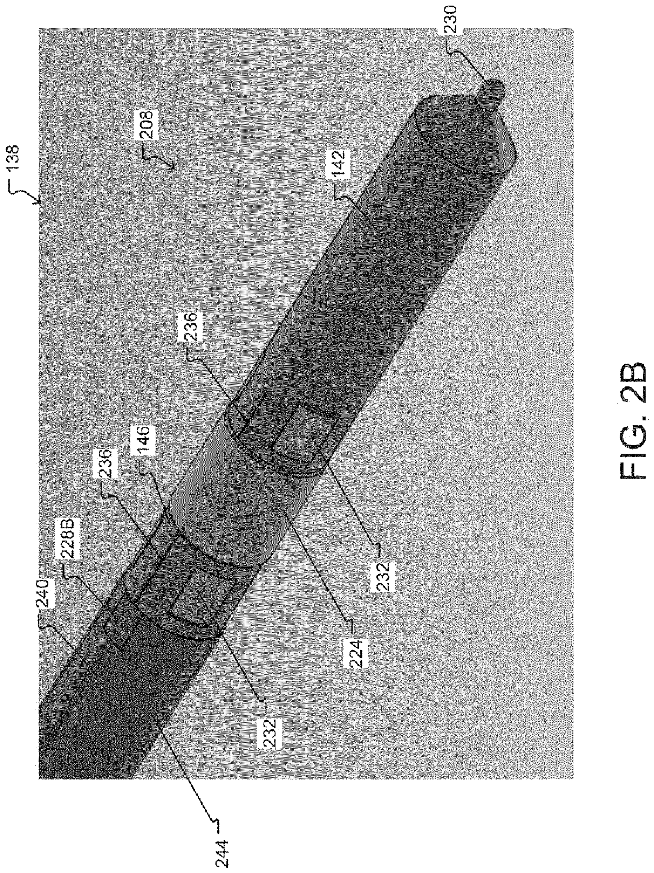

Fig. 2B illustrates a detailed perspective view of the distal end 208 of the surgical tool 138. The distal end 208 comprises the active electrode 142, the return electrode 146, a thermal bridge 224, and one or more thermocouples 228.

In some embodiments, the active electrode 142 may be the distal end electrode while the return electrode 146 may be the proximal end electrode. In other words, the active electrode 142 may be closer to the patient, the surgical site, or otherwise further away from the proximal end of the surgical tool 138 than the return electrode 146. In other embodiments, the active electrode 142 may be the proximal end electrode while the return electrode 146 may be the distal end electrode. In some embodiments, the active electrode 142 and/or the return electrode 146 may be or comprise RF electrodes (e.g., electrodes that conduct RF energy), while in other embodiments, the active electrode 142 and/or the return electrode 146 may be or comprise different materials (e.g., gold, platinum-iridium, etc.) capable of conducting different amounts of current and/or creating different lesion sizes during ablation of the anatomical tissue.

The thermal bridge 224 may be or comprise thermally conductive material (e.g., alumina ceramic, artificial diamond, combinations thereof, etc.) and/or combinations of materials enabling the thermal bridge 224 to conduct heat. The thermal bridge 224 may be disposed between the active electrode 142 and the return electrode 146, such that the thermal bridge 224 electrically isolates the active electrode 142 and the return electrode 146. In other words, the presence of the thermal bridge 224 (which is electrically insulative) prevents current from flowing directly from the active electrode 142 to the return electrode 146 (or vice versa), enabling the current to instead flow through the anatomical tissue. In some embodiments, the thermal bridge 224 may be affixed or attached to each of the active electrode 142 and the return electrode 146, such that heat generated within each of the active electrode 142 and the return electrode 146 due to current flow therethrough may be absorbed by the thermal bridge 224. In other words, the thermal bridge 224 may operate a heat sink that transfers heat generated in the active electrode 142 and/or the return electrode 146 into a coolant to dissipate heat from the surgical tool 138. The heat in the thermal bridge 224 may be removed using the fluid conduits 154 (e.g., the coolant from the inflow tube 212 absorbs the heat from the thermal bridge 224 and is pumped out by the outflow tube 216).

Different embodiments may utilize different attachment mechanisms to connect the thermal bridge 224 to the active electrode 142 and to the return electrode 146. For instance, the thermal bridge 224 may be connected to the active electrode 142 and/or the return electrode 146 using interlocking keys 232 and/or welding slots 236. The interlocking keys 232 may be made of or comprise similar or the same material as the thermal bridge 224 (e.g., alumina, artificial diamond, combinations thereof, etc.) and may slot into the active electrode 142 and/or the return electrode 146 to couple the thermal bridge 224 thereto. In some embodiments, the interlocking keys 232 and the thermal bridge 224 may be formed as a single piece capable of interfacing with the active electrode 142 and the return electrode 146. In some embodiments, the interlocking keys 232 may extend underneath the surface of the active electrode 142 and/or the return electrode 146, such that the surface area between the thermal bridge 224 and the active electrode 142 and/or return electrode 146 is increased. In other words, in addition to the thermal bridge 224 abutting the active electrode 142 and the return electrode 146, portions of the thermal bridge 224 may overlap with portions of the active electrode 142 and/or the return electrode 146. The increased surface area contact may increase the amount of heat the thermal bridge 224 can remove from the electrodes 142, 146. Additionally or alternatively, the thermal bridge 224 may be soldered to the active electrode 142 and/or the return electrode 146. In such embodiments, the solder may also be thermally conductive or may enable the conduction of heat, such that the thermal bridge 224 can absorb heat from the active electrode 142 and/or the return electrode 146. In some embodiments, the active electrode 142, the thermal bridge 224, and the return electrode 146 may be substantially cylindrical, such that, once the thermal bridge 224 is coupled to the active electrode 142 and to the return electrode 146, the combination may form a substantially flush outer surface. In such embodiments, the active electrode 142, the thermal bridge 224, and the return electrode 146 may be coaxially aligned such that the distal end 208 of the surgical tool 138 can uniformly enter the surgical site. Similarly, the active electrode 142, the thermal bridge 224, and the return electrode 146 may all share similar or the same radii.

The thermocouples 228 may include a distal thermocouple 228A and a proximal thermocouple 228B. In some embodiments, the distal thermocouple 228A and the proximal thermocouple 228B may be similar to the previously-described thermocouples 150. The distal thermocouple 228A and/or the proximal thermocouple 228B may be or comprise a type B thermocouple, a type C thermocouple, a type E thermocouple, a type J thermocouple, a type K thermocouple, a type N thermocouple, a type R thermocouple, or a type S thermocouple. In one embodiment, both the distal thermocouple 228A and the proximal thermocouple 228B comprise a type K thermocouple.

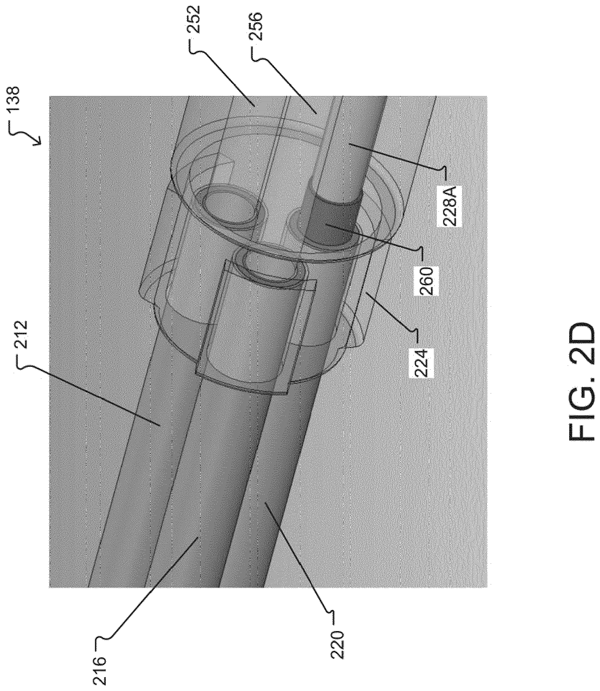

The distal thermocouple 228A may be disposed within and contact a tip 230 or other portion of the return electrode 146, such that the distal thermocouple 228A can measure the temperature of the active electrode 142. The tip 230 may be domed (e.g., spherically-shaped) and may be or comprise a conductive material (e.g., one or more conductive metals such as stainless steel), such that the tip 230 can act as an active RF electrode (or a portion thereof). In some embodiments, the tip 230 may be connected to the active electrode 142 (e.g., welded to the active electrode 142), such that the tip 230 can act as an extension of the active electrode 142 for the purposes of generating ablation using RF energy. In such embodiments, the distal thermocouple 228A may be concentrically and/or coaxially aligned within the electrical conduit 220, as illustrated in Fig. 2D, such that the distal thermocouple 228A can measure the temperature of the active electrode 142 (or components thereof such as the tip 230). In some embodiments, the distal thermocouple 228A may be or comprise an active RF electrode wire (not shown) that may run from the proximal end 204 of the surgical tool 138 through the electrical conduit 220 that contacts or is otherwise in electrical communication with the tip 230 and/or the active electrode 142. The active RF electrode wire may conduct current from an energy source (e.g., a battery, a generator, etc.) into the tip 230 and/or the active electrode 142 to enable RF ablation of anatomical tissue.

While the distal thermocouple 228A may be disposed within the tip 230 mechanically coupled with the active electrode 142, the proximal thermocouple 228B may be disposed proximate the return electrode 146. In some embodiments, the proximal thermocouple 228B may directly abut the return electrode 146 such that the proximal thermocouple 228B can measure the temperature of the return electrode 146 during operation of the surgical tool 138. The thermocouples 228 may generate separate temperature measurements that may be provided (wired and/or wirelessly) to the computing device 102. Due to the coaxially nature of the active electrode 142, the thermal bridge 224, and the return electrode 146, the proximal thermocouple 228B may be disposed near the surface of the surgical tool 138 and/or directly proximate the return electrode 146 without interfering with the flow of coolant through the surgical tool 138 and without interfering with the current flow to the active electrode 142.

The proximal thermocouple 228B may include a return wire 240. The return wire 240 may be a current-conducting wire that runs from the proximal thermocouple 228B to the proximal end 204 of the surgical tool 138 to carry the current flowing through the electrical conduit 220, the active electrode 142, the anatomical tissue, and the return electrode 146 back to the power source (e.g., a battery, a generator, etc.) to complete the circuit. In some embodiments, the proximal thermocouple 228B may be disposed between the return wire 240 and the return electrode 146. The return wire 240 may be insulated with insulation 244. The insulation 244 serves to electrically insulate the return 240 from other components in the surgical tool 138. The insulation 244 may extend partially or completely down the length of the elongated shaft 206 to the proximal end 204 of the surgical tool 138, such that the return wire 240 is insulated up to the power source. The insulation 244 may be or comprise one or more insulative materials (e.g., glass, plastic, porcelain, etc.) that prevent the current conducted by the return wire 240 from passing through other components of the surgical tool 138. In one embodiment, the insulation 244 may be or comprise polyethylene terephthalate (PET). In some embodiments, the insulation 244 may be wrapped partially or completely around the proximal thermocouple 228B, the return wire 240, and/or the elongated shaft 206. In one embodiment, the insulation 244 may surround the elongated shaft 206 such that the insulation 244 remains flush with the active electrode 142, with the proximal thermocouple 228B embedded in a portion of the insulation 244.

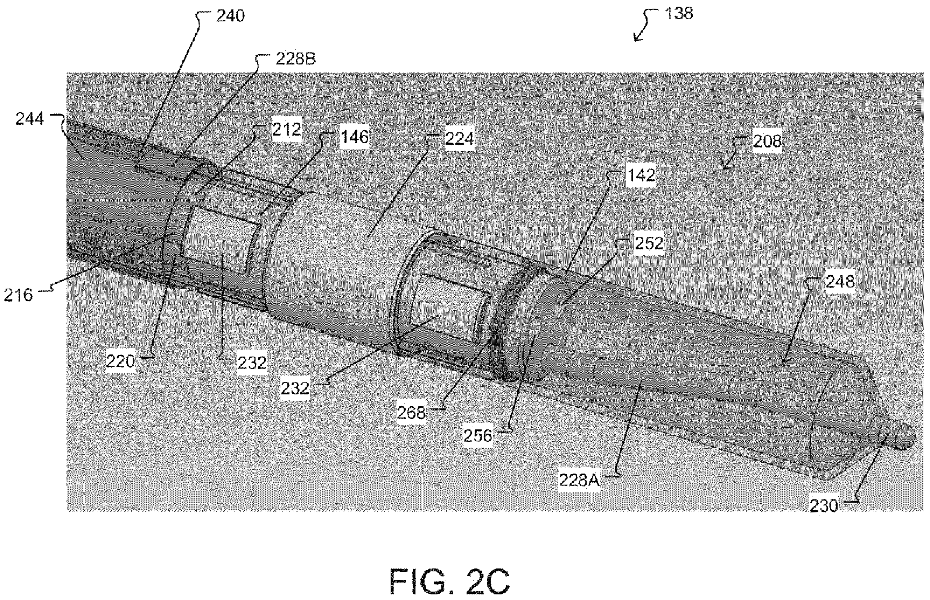

Fig. 2C shows aspects including an interior of the surgical tool 138 according to at least one embodiment of the present disclosure. The active electrode 142 includes an interior cavity 248. Both the interior cavity 248 and the active electrode 142 may contact the thermal bridge 224. The interior cavity 248 may permit the electrical conduit 220 to pass through the thermal bridge 224 and conduct current into the active electrode 142. The distal thermocouple 228A may terminate at the tip 230 of the active electrode 142. The connection of the tip 230 with the active electrode 142 may allow current to flow from the active RF carrier wire (not shown) within the distal thermocouple 228A to the tip 230 and out of the active electrode 142 and into anatomical tissue associated with the patient.

The thermal bridge 224 may contain an inflow channel 252 and outflow channel 256. The inflow channel 252 may be fluidically connected to the inflow tube 212, such that coolant can flow into the interior cavity 248 to cool the active electrode 142. Similarly, the outflow channel 256 may be fluidically connected to the outflow tube 216 such that the coolant can be extracted from the interior cavity 248. In some embodiments, and as shown in Fig. 2D, the inflow tube 212 and/or the outflow tube 216 may respectively interface with the inflow channel 252 and/or the outflow channel 256 in an interior of the thermal bridge 224, such that the inflow tube 212 and/or the outflow tube 216 does not extend into the interior cavity 248. Similarly, the electrical conduit 220 may end within the thermal bridge 224, while the distal thermocouple 228A and/or the active RF wire may extend through the thermal bridge 224 into the interior cavity 248 and up to the tip 230. In other embodiments, the electrical conduit 220 may continue through the interior of the thermal bridge 224, into the interior cavity 248, and up to the tip 230.

In some embodiments, insulation 260 may also end within the interior of the thermal bridge 224, such that the insulation 260 does not extend into the interior cavity 248 of the active electrode 142. The insulation 260 may be similar to or the same as the insulation 244, and may partially or wholly surround the active RF wire and/or the distal thermocouple 228A within the electrical conduit 220. The insulation 260 may create an insulative shielding of the active RF wire to mitigate or prevent capacitive coupling (which may contribute to tube heating, energy loss, inconsistent power delivery to the anatomical tissue and/or proximal locations outside the lesion zone, etc.) between the active RF electrode and one or more other components of the surgical tool 138 (e.g., the return electrode 146).

The interior cavity 248 may include an O-ring 268. The O-ring 268 may be disposed around a portion of the thermal bridge 224 contained within and/or contacting the interior cavity 248 to prevent or mitigate fluid loss. That is, the interior cavity 248 may overlap the thermal bridge 224, such that gaps between the interior cavity 248 and the thermal bridge 224 may permit the coolant flowing into and out of the interior cavity 248 to leak out of the interior cavity 248. To prevent coolant leaks, the O-ring 268 may be disposed around portions the thermal bridge 224 and/or the interior cavity 248. The O-ring 268 may be or comprise one or more stretchable and/or insulative materials (e.g., Polytetrafluoroethylene (PTFE), Nitrile, Neoprene, EPDM Rubber, combinations thereof, and/or the like) capable of stretching or otherwise fitting around the thermal bridge 224.

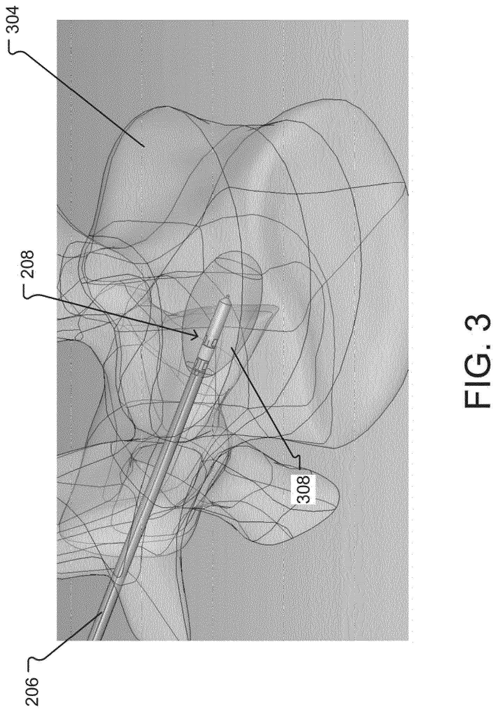

Fig. 3 shows a possible application of a surgical tool 138 to a surgical site of an anatomical element 304. The anatomical element 304 may be or comprise a vertebra (e.g., a lumbar vertebra, a cervical vertebra, a thoracic vertebra, a sacral vertebra). Notwithstanding the foregoing, the anatomical element 304 may be or comprise different anatomical elements (e.g., other bones, other soft tissues, etc.) than the vertebrae discussed herein.

The anatomical element 304 may be accessed by the surgical tool 138. For example, a pilot hole followed by a surgical drilling may permit the surgical tool 138 to access the surgical site. More specifically, the distal end 208 and elongated shaft 206 may be inserted into the hole in the anatomical element 304, such that the distal end 208 contacts or is in proximity to the surgical site. The surgical tool 138 may then be used to ablate the anatomical tissue in the anatomical element 304. As the ablation occurs, a lesion 308 may be formed in the anatomical tissue. The lesion 308 may be or comprise ablated anatomical tissues. The size and shape of the lesion 308 may vary depending on, for example, the amount of current flowing through the anatomical tissue, the coolant flow rate in the surgical tool 138, the positioning of the distal end 208 of the surgical tool 138 relative to the anatomical tissue, combinations thereof, and/or the like. After the lesion 308 has been formed, the elongated shaft 206 and/or the distal end 208 may be extracted from the surgical site or, more generally, from the anatomical element.

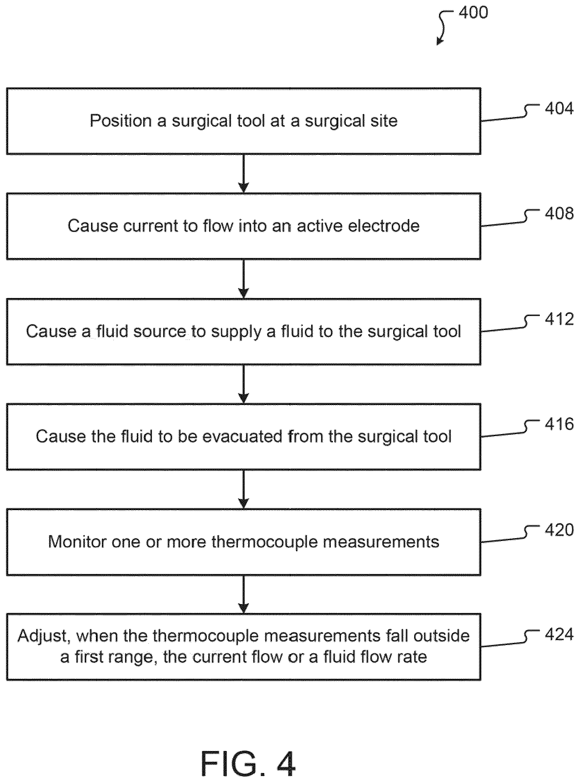

Fig. 4 depicts a method 400 that may be used, for example, to ablate anatomical tissue using a surgical tool.

The method 400 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 400. The at least one processor may perform the method 400 by executing elements stored in a memory such as the memory 106. The elements stored in memory and executed by the processor may cause the processor to execute one or more steps of a function as shown in method 400. One or more portions of a method 400 may be performed by the processor executing any of the contents of memory, such as an image processing 120, a comparison 122, a transformation 124, and/or a registration 128.

The method 400 comprises positioning a surgical tool at a surgical site (step 404). The positioning may include viewing a live video feed (e.g., an ultrasound feed) of an anatomical element (e.g., anatomical element 304) in the surgical site that is to be subjected to a surgery or surgical procedure (e.g., an ablation) and the surgical tool (e.g., the surgical tool 138) as the surgical tool is positioned relative to the anatomical element to perform the surgery or surgical procedure. In some embodiments, the surgical tool may be positioned autonomously or semi-autonomously by a robotic arm (e.g., a robotic arm 116).

The method 400 also comprises causing current to flow into an active electrode (step 408). Once the surgical tool is positioned correctly, the surgical tool may begin to operate. The current may be generated by a power source (e.g., a battery, a generator, etc.), and may flow through the active RF wire within an electrical conduit (e.g., electrical conduit 220) into the distal end of the surgical tool. Due to the configuration of the surgical tool, the current flowing through the distal end may exit into an active electrode, travel through the anatomical tissue, and out through a return electrode. The movement of the RF energy through the anatomical tissue may cause the formation of a lesion.

The propagation of current through the surgical tool may be controlled by, for example, a computing device (e.g., computing device 102). In other embodiments, a user (e.g., a surgeon, a member of surgical staff, etc.) may control the flow of current through the surgical tool.

The method 400 also comprises causing a fluid source to supply a fluid to the surgical tool (step 412). As the surgical tool operates, a fluid management system (e.g., a fluid reservoir, pumps, fluid conduits, and a control such as the computing device 102) may pump a coolant (e.g., water, sterilized water, saline, etc.) into the surgical tool. The coolant may flow into the surgical tool through an inflow tube, and may be dispensed into a cavity of the distal end of the surgical tool.

The method 400 also comprises causing the fluid to be evacuated from the surgical tool (step 416). The coolant may then be pumped out of the cavity through an outflow tube back to the fluid reservoir. The coolant may draw heat from a heat sink or thermal bridge positioned between the active and return electrodes, with the heat sink or thermal bridge conducting heat out of the electrodes and into the coolant.

The method 400 also comprises monitoring one or more thermocouple measurements (step 420). The one or more thermocouples may be positioned proximate to the active electrode and/or the return electrode and may be configured to generate a temperature measure of the active electrode, the return electrode, and/or of the surrounding anatomical tissue (e.g., the lesion being formed). The measurements may be reflective of the overall operating health of the surgical tool, as well as the health of the anatomical tissue being ablated. For instance, temperature measurements that are too high (e.g., relative to a baseline or anticipated reading) may indicate that the anatomical tissue is being charred.

The method 400 also comprises adjusting, when the thermocouple measurements fall outside a first range, the current flow or a fluid flow rate (step 424). The step 424 may implement a comparison (e.g., comparison 122) to compare the thermocouple measurements to baseline, anticipated, or otherwise predetermined measurements. When the thermocouple measurements fall outside the first range, this may indicate that the surgical tool is not functioning optimally (e.g., the temperature values are too high which may indicate that the anatomical tissue is being charred, the temperature values are too low which may indicate that the surgical tool is not ablating the anatomical tissue, etc.). In such cases, the computing device may generate an alert (e.g., a visual alert rendered to a display, an alarm, etc.) and/or adjust the operating parameters of the surgical tool. For example, the computing device may increase or decrease the current flow (e.g., to generate more or less RF energy to adjust the ablation of the anatomical tissue), and/or increase or decrease the coolant flow rate (e.g., increasing coolant flow rate to remove excess heat). In some embodiments, the computing device may continually monitor the thermocouple measurements throughout the ablation process, and provide adjustments to the operating parameters of the surgical tool when the thermocouple readings fall outside the predetermined range.

The present disclosure encompasses embodiments of the method 400 that comprise more or fewer steps than those described above, and/or one or more steps that are different than the steps described above.

As noted above, the present disclosure encompasses methods with fewer than all of the steps identified in Fig. 4 (and the corresponding description of the method 400), as well as methods that include additional steps beyond those identified in Fig. 4 (and the corresponding description of the method 400). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

Further disclosed herein is the subject-matter of the following clauses:
1. A surgical tool, comprising:
   a proximal end; and
   a distal end, the distal end comprising:
      a first electrode;
      a second electrode different from the first electrode; and
      a thermal bridge that is disposed between the first electrode and the second electrode and that electrically isolates the first electrode from the second electrode.
2. The surgical tool of clause 1, wherein the distal end further comprises:
   a thermocouple disposed on a proximal end of the first electrode.
3. The surgical tool of clause 1 or 2, wherein the first electrode comprises an active Radio Frequency (RF) electrode, and wherein the second electrode comprises a return RF electrode.
4. The surgical tool of clause 1 or of any of the preceding clauses, wherein the thermal bridge is at least one of alumina or artificial diamond.
5. The surgical tool of clause 1 or of any of the preceding clauses, wherein the first electrode, the second electrode, and the thermal bridge are substantially cylindrical, and wherein the first electrode, the second electrode, and the thermal bridge are aligned coaxially.
6. The surgical tool of clause 5 or of any of the preceding clauses, further comprising:
   an inflow tube disposed in an interior of the surgical tool and extending from the proximal end to a first distance from the distal end, the inflow tube configured to discharge a coolant into a cavity in the distal end of the surgical tool; and
   an outflow tube disposed in the interior of the surgical tool and extending from the proximal end to the first distance from the distal end, the outflow tube configured to remove the coolant from the cavity in the distal end of the surgical tool.
7. The surgical tool of clause 6 or of any of the preceding clauses, wherein the first distance from the distal end is within an interior of the thermal bridge.
8. The surgical tool of clause 1 or of any of the preceding clauses, wherein the thermal bridge comprises one or more interlocking keys, and wherein the one or more interlocking keys connect the thermal bridge to the first electrode and to the second electrode.
9. The surgical tool of clause 1 or of any of the preceding clauses, wherein the thermal bridge is soldered to at least one of the first electrode or the second electrode.
10. The surgical tool of clause 1 or of any of the preceding clauses, wherein the second electrode comprises a domed tip, and wherein the surgical tool further comprises:
   an active RF wire extending through an interior of the surgical tool and connected to the domed tip.
11. A surgical system, comprising:
   a cylindrical housing having a proximal end and a distal end;
   a proximal tube, a first end of the proximal tube connected to a first end of the distal end;
   a first thermocouple disposed between at least a portion of a return wire and the proximal tube;
   a heat sink, a first end of the heat sink connected to a second end of the proximal tube; and
   a distal tube, a first end of the distal tube connected to a second end of the heat sink.
12. The surgical system of clause 11, further comprising:
   an inflow conduit disposed in the cylindrical housing and configured to dispense a coolant into a cavity within the distal tube; and
   an outflow conduit disposed in the cylindrical housing and configured to extract the coolant from the cavity within the distal tube.
13. The surgical system of clause 11 or 12, wherein at least one of a first end of the inflow conduit or a first end of the outflow conduit is disposed in the heat sink, and wherein the cylindrical housing comprises an air gap between the cavity and the distal tube.
14. The surgical system of clause 13 or of any of the clauses 11 to 13, wherein the surgical system further comprises:
   a polyimide insulation layer disposed around at least one of the cylindrical housing or the return wire.
15. The surgical system of clause 14 or of any of the clauses 11 to 14, wherein the heat sink comprises at least one of alumina or artificial diamond.
16. The surgical system of clause 14 or of any of the clauses 11 to 15, wherein the cylindrical housing further comprises:
   an active Radiofrequency (RF) wire extending from the proximal end of the cylindrical housing to a domed tip disposed in the distal tube, the active RF wire configured to carry current to the distal tube.
17. The surgical system of clause 16 or of any of the clauses 11 to 16, wherein the heat sink is soldered to at least one of the proximal tube or the distal tube.
18. An apparatus, comprising:
   a fluid management system including an inflow tube and an outflow tube; and
   a surgical probe, the surgical probe comprising:
      an active Radiofrequency (RF) electrode;
      a return RF electrode; and
      a distal bridge that physically and thermally couples the return RF electrode and the active RF electrode and electrically decouples the return RF electrode and the active RF electrode.
19. The apparatus of clause 18, wherein at least one of the inflow tube and the outflow tube is in fluidic communication with the surgical probe, and wherein at least one of the inflow tube and the outflow tube ends within the distal bridge.
20. The apparatus of clause 18 or 19, wherein the distal bridge comprises at least one of alumina or artificial diamond, and wherein the surgical probe further comprises:
   a thermocouple disposed on a proximal end of the return RF electrode, wherein the thermocouple generates a temperature reading of the return RF electrode.

## Claims

1. A surgical tool, comprising:
a proximal end; and
a distal end, the distal end comprising:
a first electrode;
a second electrode different from the first electrode; and
a thermal bridge that is disposed between the first electrode and the second electrode and that electrically isolates the first electrode from the second electrode.

2. The surgical tool of claim 1, wherein the distal end further comprises:
a thermocouple disposed on a proximal end of the first electrode,
and/or
wherein the first electrode comprises an active Radio Frequency (RF) electrode, and wherein the second electrode comprises a return RF electrode.

3. The surgical tool of one of the claims 1 or 2, wherein the thermal bridge is at least one of alumina or artificial diamond.

4. The surgical tool of one of the claims 1 to 3, wherein the first electrode, the second electrode, and the thermal bridge are substantially cylindrical, and wherein the first electrode, the second electrode, and the thermal bridge are aligned coaxially.

5. The surgical tool of one of the claims 1 to 4, further comprising:
an inflow tube disposed in an interior of the surgical tool and extending from the proximal end to a first distance from the distal end, the inflow tube configured to discharge a coolant into a cavity in the distal end of the surgical tool; and
an outflow tube disposed in the interior of the surgical tool and extending from the proximal end to the first distance from the distal end, the outflow tube configured to remove the coolant from the cavity in the distal end of the surgical tool,
particularly wherein the first distance from the distal end is within an interior of the thermal bridge.

6. The surgical tool of one of the claims 1 to 5, wherein the thermal bridge comprises one or more interlocking keys, and wherein the one or more interlocking keys connect the thermal bridge to the first electrode and to the second electrode, and/or
wherein the thermal bridge is soldered to at least one of the first electrode or the second electrode.

7. The surgical tool of one of the claims 1 to 6, wherein the second electrode comprises a domed tip, and wherein the surgical tool further comprises:
an active RF wire extending through an interior of the surgical tool and connected to the domed tip.

8. A surgical system, comprising:
a cylindrical housing having a proximal end and a distal end;
a proximal tube, a first end of the proximal tube connected to a first end of the distal end;
a first thermocouple disposed between at least a portion of a return wire and the proximal tube;
a heat sink, a first end of the heat sink connected to a second end of the proximal tube; and
a distal tube, a first end of the distal tube connected to a second end of the heat sink.

9. The surgical system of claim 8, further comprising:
an inflow conduit disposed in the cylindrical housing and configured to dispense a coolant into a cavity within the distal tube; and
an outflow conduit disposed in the cylindrical housing and configured to extract the coolant from the cavity within the distal tube, particularly
wherein at least one of a first end of the inflow conduit or a first end of the outflow conduit is disposed in the heat sink, and wherein the cylindrical housing comprises an air gap between the cavity and the distal tube.

10. The surgical system of one of the claims 8 to 9, wherein the surgical system further comprises:
a polyimide insulation layer disposed around at least one of the cylindrical housing or the return wire.

11. The surgical system of one of the claims 8 to 10, wherein the heat sink comprises at least one of alumina or artificial diamond, and/or
wherein the cylindrical housing further comprises:
an active Radiofrequency (RF) wire extending from the proximal end of the cylindrical housing to a domed tip disposed in the distal tube, the active RF wire configured to carry current to the distal tube.

12. The surgical system of one of the claims 8 to 11, wherein the heat sink is soldered to at least one of the proximal tube or the distal tube.

13. An apparatus, comprising:
a fluid management system including an inflow tube and an outflow tube; and
a surgical probe, the surgical probe comprising:
an active Radiofrequency (RF) electrode;
a return RF electrode; and
a distal bridge that physically and thermally couples the return RF electrode and the active RF electrode and electrically decouples the return RF electrode and the active RF electrode.

14. The apparatus of claim 13, wherein at least one of the inflow tube and the outflow tube is in fluidic communication with the surgical probe, and wherein at least one of the inflow tube and the outflow tube ends within the distal bridge.

15. The apparatus of one of the claims 13 or 14, wherein the distal bridge comprises at least one of alumina or artificial diamond, and wherein the surgical probe further comprises:
a thermocouple disposed on a proximal end of the return RF electrode, wherein the thermocouple generates a temperature reading of the return RF electrode.
